# EUROPÄISCHE PATENTSCHRIFT

(11) **EP 0 374 910 B1**
(45) Veröffentlichungstag und Bekanntmachung des Hinweises auf die Patenterteilung: **07.07.1993**
(21) Anmeldenummer: 89123619.2
(22) Anmeldetag: 21.12.1989
(51) Int. Cl.: A61F 13/15

(54) **Hochsaugaktive Vliesstoffbahn**
Highly absorbent bonded fabric web
Bande d'étoffe nappée de haute capacité d'absorption

(30) Priorität: 21.12.1988 DE 8815855 U
(43) Veröffentlichungstag der Anmeldung: 27.06.1990
(73) Patentinhaber: Steen & Co. GmbH, D-22508 Hamburg (DE)
(72) Erfinder: Knack Ingo, Dr., D-2053 Schwarzenbek (DE)
(74) Vertreter: Lederer, Franz, Dr.

(56) Entgegenhaltungen:
- FR-A- 2 350 830
- FR-A- 2 404 996
- GB-A- 1 192 581
- US-A- 3 901 236

## Beschreibung

Gegenstand der vorliegenden Erfindung ist eine hochsaugaktive Vliesstoffbahn bestehend aus einem zwischen zwei Deckvliesschichten eingebetteten hochsaugaktiven Saugkörper.

Aus dem Stand der Technik sind Laminatprodukte bekannt, die flüssigkeitsabsorbierende Komponenten enthalten. Beispielsweise wird in der DE-OS 35 11 805 ein Hygieneartikel beschrieben, der ein Kernmaterial umfaßt, bei welchem ein wasserabsorbierendes Polymeres von zwei Schichten aus Zellstoff-Flocken eingeschlossen ist, die das Polymere umgeben. Dabei wird als Hauptabsorptionsmittel eine aus Zellstoff-Flocken bestenende zusammengepreßte Schicht verwendet. Zwischen die auf eine spezielle Weise zusammengedrückten Zellstoff-Flockenschichten wird wasserabsorbierendes Polymeres eingeschlossen. Damit das Polymere zwischen den beiden Schichten aus Zellstoff-Flocken eingeschlossen bleibt, werden bestimmte Anforderungen an die Geometrie der Schichten aus Zellstoff-Flocken gestellt.

In der GB-PS 1 192 581 werden absorbierende Gegenstände wie Tampons etc. beschrieben, die ein Absorptionsmaterial innerhalb von Begrenzungsschichten aufweisen. Als Saugkörper wird unter anderem vernetztes Polyacrylamid offenbart, das allerdings nicht in Form von Faserflocken, die ein anderes Polymer aufweisen, vorliegt.

Es hat sich herausgestellt, daß bei der Einarbeitung von pulverförmigen Absorbern (Polymeren) zwischen eine Grund- und eine Deckschicht nachteilige Eigenschaften erhalten werden.

Da die zur Weiterverarbeitung dienenden Ausgangsmaterialien häufig als Rollenware geliefert werden, aus der die entsprechend gestalteten Teile ausgeschnitten werden, kommt es häufig vor, daß die pulverförmigen Absorptionsmaterialien zumindest in der Nähe des Schnittbereiches herausrieseln. Dadurch tritt ein Verlust an Absorptionsmaterial auf und der herausrieselnde Staub führt zu erschwerten Arbeitsbedingungen bei den Herstellungsverfahren. Darüberhinaus ist der Gehalt an Absorptionsmaterial in der Gegend des Schnittbereiches vermindert.

Ein weiterer Nachteil der pulverförmige Absorptionsmaterialien enthaltenden Laminatprodukte ist, daß nur eine relativ geringe Menge des pulverförmigen wasserabsorbierenden Polymermaterials eingearbeitet werden kann, da anderenfalls bei Herstellungsverfahren, bei Lagerung oder Transport eine unerwünschte räumliche Umorientierung der pulverförmigen Polymeren verursacht wird. Die pulverförmigen Absorptionsmaterialien können sich dann an einer Stelle im fertigen Produkt sammeln, was einerseits zu mechanischen Beeinträchtigungen des Produktes führen kann (Rißbildung), und andererseits die Handhabung des fertigen Gebrauchsgegenstandes durch den Verbraucher beeinträchtigen kann.

Aus der US-PS 3 901 236 sind Faserflocken bekannt, die einen Saugkörper aufweisen. Bevorzugt verwendet werden dort allerdings hydrophile Fasern, wie Cellulosefasern.

Erfindungsgemäß wird nun eine saugaktive Vliesstoffbahn zur Verfügung gestellt, die die oben genannten Nachteile nicht aufweist. Die hochsaugaktive Vliesstoffbahn besteht aus zwei, im wesentlichen parallel zueinander angeordneten Deckvliesschichten, die einen zur Speicherung wäßriger Flüssigkeiten fähigen Saugkörper umschließen. Teilweise sind die Deckvliesstoffe miteinander verbunden. Die Verbindungsbereiche können entweder nur an den Rändern und/oder über die gesamte Fläche des Vliesstoftes verteilt angeordnet sein. Bevorzugt werden die Deckvliesschichten so angeordnet, daß rechteckige oder quadratische Kompartimente entstehen.

Wesentlich ist, daß der Saugkörper, der zwischen den Deckvliesschichten angeordnet ist, aus einer Schicht von Faserflocken besteht. Die einzelnen Faserflocken werden im folgenden auch als Superabsorberfaserflocken bezeichnet.
Die erfindungsgemäß verwendeten Superabsorberfaserflocken bestehen aus einem Kern aus einem saugfähigen Polymer in den kurze Polyolefinfasern eingelagert sind, die zumindest teilweise aus dem Kern herausragen.

Bei der bevorzugten Ausführungsform besteht bei den erfindungsgemäß verwendeten Superabsorberfaserflocken der Kern aus mittels eines Haftvermittlers miteinander verbundenen Partikeln des saugfähigen Polymers und den Polyolefinfasern.

Das erfindungsgemäß verwendete saugfähige Polymermaterial muß in der Lage sein, eine möglichst große Menge einer wäßrigen Flüssigkeit absorbieren zu können. In der Regel werden dazu bevorzugt Polymerisate auf der Basis von Acrylsäure/Natriumacrylat verwendet. Aber auch andere Polymerisate auf der Grundlage von vernetzten Polymeren können im Rahmen der vorliegenden Erfindung verwendet werden. Beispiele für derartige Polymere sind quervernetzte Polysaccharide, wie beispielsweise Carboxymethylcellulose oder Stärkeether, und anorganische, poröse Materialien, wie beispielsweise Zeolithe.

In der Regel muß das verwendete Polymermaterial ein Mehrfaches seines Gewichtes an Wasser aufnehmen können; das bevorzugt verwendete Acrylsäure/Natriumacrylat-Polymer kann dabei mindestens die dreifache Menge seines Gewichtes an Wasser aufnehmen, wobei das Polymer in einen gelartigen Zustand übergeht.

Ein im Rahmen der vorliegenden Erfindung besonders bevorzugtes Polyacrylat-Produkt ist das im Handel erhältliche FAVOR^{®} SAB 922 (Firma Stockhausen). Es weist die folgenden chemischen Daten auf:

| | |
|---|---|
| Chemische Basis: | Natrium-Polyacrylat |
| Beschaffenheit: | weißes Granulat |
| Korngröße: | 100-630 Mikron |
| Schüttgewicht: | 690 ± 30 g/l |
| Rieselverhalten: | frei fließend |
| Trockensubstanz: | 95 ± 2 % |
| pH-Wert: | 6,0 ±1 |
| (0,1 % Gel in 0,9 % NaCl) | |
| Lagerung: | bei trockener Lagerung 1 Jahr |

Dieses Natrium-Polyacrylat ist ein schwach vernetztes Polymerisat auf der Basis Acrylsäure/Natriumacrylat.

Dieses bevorzugte Polymermaterial quillt bei Wasseraufnahme zu einem weitgehend noch trockenen und körnigen Gel ohne zu zerfallen.

Als Polyolefinfasern werden erfindungsgemäß bevorzugt Polyethylen (PE-) und Polypropylen (PP-) -Kurzschnittfasern verwendet. Diese Fasern werden nach an sich bekannten Verfahren hergestellt und weisen bevorzugt eine Länge zwischen 1 mm und 6 mm und besonders bevorzugt zwischen 2 mm und 4 mm auf. Im allgemeinen weisen die Fasern einen Titer von 0,5-6 dtex auf, wobei ein Titer von 2-4 dtex bevorzugt wird. Eine leichte Längenabweichung der einzelnen Fasern Stört bei der neuerungsgemäßen Verwendung nicht. Es kann sogar vorteilhaft sein, gezielt unterschiedliche Faserlängen einzusetzen, um bevorzugte Haftwirkungen des Polymerproduktes in der Fluffschicht zu erzielen.

Im Rahmen der vorliegenden Erfindung sind die genannten PE- und PP-Kurzschnittfasern bevorzugt, da diese Fasern aufgrund ihrer molekularen Struktur eine Dochtwirkung entfalten, so daß sie die Flüssigkeit von dem Entstehungsort zu den hochsaugfähigen Polymerprodukten weiterleiten können. Zur Verbesserung der Haftfähigkeit des faserhaltigen hochsaugaktiven Polymerproduktes kann es vorteilhaft sein, die Fasern einer speziellen Behandlung zu unterziehen, durch die die Struktur der Fasern derart verändert wird, daß diese leicht gekräuselt sind und somit eine bessere mechanische Interaktion mit den Fasern der Matrix-Struktur, in die die erfindungsgemäßen Polymerpartikel eingebettet sind, zu erzielen. Ein derartiger zusätzlicher Behandlungsschritt kann das Recken der Fasern vor dem entsprechenden Ablängen sein. Daraus kann eine Flockenschicht geformt werden, die den Saugkörper bildet.

Zur Herstellung der erfindungsgemäß verwendeten Faserflocken können prinzipiell zwei Wege beschritten werden.
a) Das Polymerprodukt mit hoher Absorptionsfähigkeit wird mit den Fasern vermischt und die Verbindung durch Zusatz eines Haftvermittlers erzielt. Die Art des verwendeten Haftvermittlers hängt sowohl von der Art des eingesetzten Polymerproduktes, wie auch von der Art der eingesetzten Fasern ab. Aufgrund der chemischen Zusammensetzung des absorptionsrähigen Polymermaterials und der Fasern kann der Fachmann diejenigen Haftvermittler auswählen, durch die eine zuverlässige Verbindung zwischen den beiden oben genannten Komponenten erzielt werden kann. Darüber hinaus dürfen die Haftvermittler natürlich keine unerwünschten Nebenwirkungen aufweisen, die von der Art der Verwendung abhängen. Sollen die Polymerprodukte beispielsweise in Hygieneartikeln, wie Windeln verwendet werden, müssen die Haftvermittler gesundheitlich unbedenklich sein.
   Unter der großen Vielzahl der bekannten Haftvermittler werden im Rahmen der vorliegenden Erfindung Ethylen/Acrylamid-Copolymere, Polymere-Isocyanate oder reaktive Silicium-organische Verbindungen bevorzugt, wobei Ethylen/Acrylsäure-Copolymere, Zelluloseether oder Polyvinylalkohole besonders bevorzugt sind.
   Die Zugabe eines Haftvermittlers kann entbehrlich sein, wenn durch die Dispersion des Polymerproduktes bereits eine Gelbildung in den Randbereichen des körnigen Polymerproduktes erzielt wird, und wenn dadurch bereits eine hinreichend feste Verbindung von Polymerprodukt und Faser erreicht werden kann.
b) Ein anderer Weg, das Fasermaterial mit dem Polymerprodukt in Verbindung zu bringen, besteht darin, daß das Fasermaterial vor der Fertigstellung des Polymermaterials in die Polymerlösung zugegeben wird und daß anschließend die Vernetzungsreaktion stattfindet. Dabei werden die Fasern, zumindest teilweise in das Polymerprodukt eingeschlossen, so daß sich eine Verbindung der Faserenden mit dem Inneren des Polymerproduktes ergibt. Durch eine derartige Herstellungsweise kann ein Produkt erzielt werden, bei dem sich die Enden der Fasern im Inneren des Polymers befinden, wodurch eine schnellere und effektivere Übermittlung der zu entfernenden Flüssigkeit in das Polymer erzielt wird, da die Fasern, wie bereits erwähnt eine Dochtwirkung aufweisen.
   Bei dieser Art der Herstellung kann eine Öl-in-Wasser-Polymerisation eingesetzt werden.

Das Verhältnis der eingesetzten Fasern zu dem hochsaugaktiven Polymerprodukt kann in relativ weiten Bereichen im allgemeinen im Bereich von 10:1 bis 1:10 verändert werden. Bevorzugt wird ein Gewichtsverhältnis von Fasern zu hochsaugaktivem Polymerprodukt von 1:1 bis 1:10 und insbesondere von 1:2 bis 1:8. Auf diese Weise lassen sich Produktvariationen gewinnen, die von einem mehr watteähnlichen, flockigen bis zu einem im wesentlichen kornartigen Polymerprodukt reichen können.

Nach dem Verkleben der Polymerkörner untereinander und mit den eingesetzten Fasern werden kleinere bis mittelgroße Faserflocken erhalten, die in einer Schlagmühle zerkleinert und anschließend getrocknet werden.

Die fertige Faserflocke enthält dann viele kleine Fasern in den einzelnen teilchenförmigen Polymerpartikeln eingeschlossen, die insbesondere mit ihren Enden aus diesen herausragen.

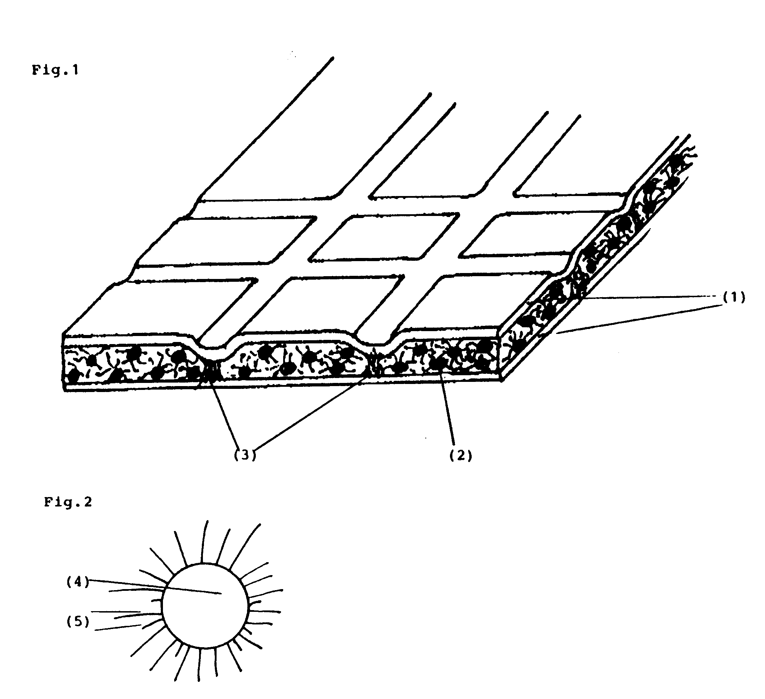

In Figur 2 wird eine erfindungsgemäß verwendete Superabsorberfaserflocke schematisch dargestellt, die aus dem Kern aus einem saugfähigen Polymer (4) und den herausstehenden Polymerfasern (5) besteht.

Es hat sich herausgestellt, daß die zur Herstellung des neuerungsgemäßen Vliesstoffes besonders geeigneten Superabsorberflocken eine Größe von 0,5 bis 2 mm aufweisen, wobei der Flockenkern eine Abmessung zwischen 0,1 und 1 mm aufweist.

Aus den Superabsorberfaserflocken können durch Aufschütten auf einfache Weise stabile Schichten hergestellt werden, die zwar nicht starr sind, jedoch an dem aufgetragenen Ort durch die Deckvliesschichten räumlich fixiert werden. Das Flächengewicht der Saugschicht kann zwischen 10 und 500 g/m² liegen. Bevorzugt werden Saugkörperschichten mit einem Flächengewicht von 20 g/m² bis 200 g/m² verwendet.

Die Superabsorberfaserflocken können zumindest zum Teil durch solche saugaktive Flocken ersetzt werden, die sich zur Bildung einer Saugkörperschicht eignen. Ein Beispiel hierfür sind Zelluloseflocken.

Dieser hochsaugaktive Saugkörper wird von zwei im wesentlichen parallel zueinander angeordneten Deckvliesschichten umschlossen. Hergestellt werden können diese Deckvliese grundsätzlich aus allen zur Vlieslegung geeigneten Fasern bzw. Faserpolymeren. Erfindungsgemäß werden bevorzugt Polyolefine zur Herstellung der Vliesstoffe verwendet. Besonders bevorzugt sind dabei Vliesstoffe aus Polyethylen und Polypropylen.

Üblicherweise wird die Dicke des verwendeten Deckvliesstoffes durch das Flächengewicht angegeben. Dieses kann zwischen 4 g/m² und 300 g/m² liegen. Bevorzugt werden jedoch Vliesstoffe mit Flächengewichten zwischen 15 und 80 g/m², wobei Flächengewichte zwischen 40 und 60 g/m² ganz besonders bevorzugt sind.

Es können aber auch andere Deckvliesstoffe verwendet werden, wobei solche Materialien bevorzugt werden, die aus einer Zelluloseschicht bestehen, auf die eine Polyolefinschicht aufgebracht wurde. Diese Deckvliesschichten haben den Vorteil, daß sie mittels einer thermischen Verfestigung miteinander verbunden werden können.

Die thermische Verfestigung kann durch Kalandrieren oder durch Heißluftanwendung erfolgen, wobei die thermoplastischen Polymere miteinander verschweißt werden. Dabei kommt es durch eine Verschweißung der Fasern der Deckvliesschichten zu einer festen Verbindung zwischen dem Saugkörper und den Deckvliesschichten, insbesondere dann, wenn die Deckvliesschichten und die Fasern der Superabsorberflocken aus Polyolefinmaterial bestehen. Bei einer besonders bevorzugten Herstellungsform werden die Deckvliesschichten und der Saugkörper kalandriert. Dabei werden die Deckvliesschichten und die Saugkörperschicht durch Walzen geführt, wobei deren Form und Gravur die Größe und Anzahl der Schweißpunkte bzw. Verbindungslinien bestimmt.

Gemäß einer anderen Ausführungsform werden die Deckvliesschichten, die aus solchen Faserpolymeren bestehen, die nicht oder nur schwer thermisch zu verfestigen sind, wie beispielsweise Viskose oder Polyester, miteinander durch chemische Binder verfestigt. Als chemische Binder können bevorzugt Copolymere aus Ethylen und Acrylsäure, Zelluloseether und Polyvinylalkohol verwendet werden.

Darüberhinaus kann die Verbindung der Deckvliesschichten und des Saugkörpers auf mechanische Weise erfolgen. Bevorzugt wird die Verfestigung durch Vernadeln erreicht.

In einer bevorzugten Ausführungsform sind die beiden Deckvliesschichten des erfindungsgemäßen hochsaugaktiven Vliesstoffes nicht nur an den Rändern, sondern auch in bestimmten Abständen über die gesamte Fläche des Vliesstoffes verteilt miteinander verbunden. Durch die Verbindung der Deckvliesschichten wird der Saugkörper in kleinere Kompartimente unterteilt. Es wird dabei eine feste Verbindung zwischen den Deckvliesschichten und dem Saugkörper an den Verbindungspunkten bzw. -linien hergestellt.

Diese Verbindungspunkte bzw. -linien können beispielsweise dadurch hergestellt werden, daß eine bestimmte Menge der Superabsorberflocken mit einem geeigneten Dosieraggregat homogen auf eine Deckvliesschicht aufgebracht und anschließend mit einer zweiten Deckvliesschicht abgedeckt wird. Die drei Lagen werden dann durch Kalandrieren verfestigt und miteinander verbunden. Durch die Wahl geeigneter Kalandrierwalzen kann die Geometrie und der Abstand der Verbindungslinien entsprechend eingestellt werden. An den Erhöhungen der Kalanderwalzen wird erhöhter Druck ausgeübt, was bei der gleichzeitigen Temperaturbehandlung zu einer festen Verbindung von Deckvliesschichten und Saugkörper führt.

Durch die Einarbeitung von Verbindungspunkten bzw. Verbindungslinien zwischen den Deckvliesschichten wird der Saugkörper in kleinere Kompartimente eingeschlossen, wobei eine feste räumliche Fixierung des Saugkörpers in den erfindungsgemäßen saugaktiven Vliesstoffen erzielt wird. Dadurch wird das Herausrieseln des Absorbermaterials an Schnittstellen verhindert, da die Fasern der Superabsorber flocken miteinander in Kontakt stehen, was eine erhöhte räumliche Fixierung bewirkt. Bei einer besonders bevorzugten Ausführungsform werden zwei Gruppen von im wesentlichen parallelen Verbindungslinien etwa im rechten Winkel zueinander so angeordnet, daß sie ein rechteckiges oder quadratisches Muster von Verbindungslinien bilden.

Die erfindungsgemäßen Vliesstoffe weisen bevorzugt ein Flächengewicht von 50 bis 250 g/m² und eine Dicke von 0,5 bis 3 mm auf.

Die erfindungsgemäßen Vliesstoffe können in weiten Bereichen verwendet werden. Sie haben die Fähigkeit wäßrige Flüssigkeiten schnell und zuverlässig zu binden. Dies ist insbesondere bei Hygieneartikeln wie Babywindeln, Damenbinden oder Inkontinenzprodukten besonders wichtig. Bei der Herstellung des erfindungsgemäßen Vliesstoffes kann bereits die spätere Verwendung berücksichtigt werden. So können bei der Verwendung des Vliesstoffes als Babywindel oder Damenbinden die Bereiche, die später einer erhöhten Flüssigkeitsbelastung ausgesetzt werden, mit einer dickeren Schicht des Saugkörpers versehen werden, wohingegen die Randbereiche, die nur eine geringere Flüssigkeitsmenge aufnehmen müssen, mit einer dünneren Schicht des Saugkörpers versehen werden können.

Weiterhin können die hochsaugaktiven Vliesstoffe auch in der Industrie angewendet werden. Häufig müssen aus organischen Lösungsmitteln oder Treibstoffen Reste von Wasser entfernt werden. Dies kann auf einfache Weise dadurch erreicht werden, daß die zu behandelnden organischen Losungsmittel durch eine oder mehrere Schichten aus dem erfindungsgemäßen Vliesstoff geleitet werden. Auch ist der Einsatz der hochsaugaktiven Vliesstoffe im Gartenbau und der Landwirtschaft zur Pflanzenbewässerung denkbar.

Figur 1 zeigt einen erfindungsgemäßen hochsaugaktiven Vliesstoff, bestehend aus zwei im wesentlichen parallel zueinander angeordneten Deckvliesschichten (1), die an Verbindungslinien (3) miteinander verbunden sind. Zwischen den Deckvliesschichten ist der aus Superabsorberflocken bestehende Saugkörper (2) angeordnet.

Figur 2 zeigt schematisch eine Superabsorberfaserflocke, die aus einem Kern aus saugfähigem Polymer (4) und den heraustehenden Polymerfasern (5) besteht.

## Patentansprüche

1. Saugaktive Vliesstoffbahn mit einem zur Speicherung wäßriger Flüssigkeiten fähigen Saugkörper, der aus einer Schicht von saugaktiven Faserflocken besteht und von zwei im wesentlichen parallel zueinander angeordneten Deckvliesschichten umschlossen ist, wobei die Deckvliesschichten teilweise miteinander verbunden sind,
dadurch gekennzeichnet, daß
die einzelnen, den Saugkörper bildenden Faserflocken aus einem kugelförmigen Kern, bestehend aus einem saugfähigem Polymer mit einem Durchmesser zwischen 0,1 und 2 mm und damit verbundenen Polyolefinfasern einer Länge zwischen 0,2 und 10 mm bestehen, wodurch eine Abmessung der einzelnen Flocke von 0,1 bis 5 mm und insbesondere von 0,5 bis 2 mm Durchmesser erzielt wird.

2. Vliesstoffbahn nach Anspruch 1, dadurch gekennzeichnet, daß das Flächengewicht der Saugschicht zwischen 20 g/m² und 200 g/m² beträgt.

3. Vliesstoffbahn nach einem der vorhergehenden Ansprüche, dadurch gekennzeichnet, daß der Vliesstoff, bestehend aus Saugkörper und Deckvliesschichten, eine Dicke von 0,2 bis 30 mm aufweist.

4. Vliesstoffbahn nach Anspruch 3, dadurch gekennzeichnet, daß der Vliesstoff eine Dicke zwischen 0,5 und 3 mm aufweist.

5. Vliesstoffbahn nach einem der vorhergehenden Ansprüche, dadurch gekennzeichnet, daß die beiden Deckvliesschichten an den Rändern und/oder in dem zwischen den Rändern liegenden Bereich durch im wesentlichen parallel zueinander angeordnete linienförmige Bereiche miteinander verbunden sind, wodurch der zwischen den Deckvliesschichten angeordnete Saugkörper in kleinere Kompartimente unterteilt wird.

6. Vliesstoffbahn nach Anspruch 5, dadurch gekennzeichnet, daß in den Verbindungsbereichen die Fasern der Deckvliesschichten miteinander verschweißt sind, wodurch eine feste Verbindung zwischen Saugkörper und den beiden Deckvliesschichten erzielt wird.

7. Vliesstoffbahn nach einem der vorhergehenden Ansprüche, dadurch gekennzeichnet, daß die Deckvliesschichten Polyolefin-Vliesschichten sind.

8. Vliesstoffbahn nach Anspruch 7, dadurch gekennzeichnet, daß das Polyolefin Polyethylen oder Polypropylen ist.

9. Vliesstoffbahn nach Anspruch 5, dadurch gekennzeichnet, daß in den Verbindungsbereichen die beiden Deckvliesschichten mit chemischen Bindern verfestigt sind.

10. Vliesstoffbahn nach Anspruch 9, dadurch gekennzeichnet, daß die chemischen Binder Copolymere aus Ethylen und Acrylsäure, Zelluloseether oder Polyvinylalkohol sind.

11. Vliesstoffbahn nach einem der vorhergehenden Ansprüche, dadurch gekennzeichnet, daß die Deckvliesschichten ein Flächengewicht von jeweils 15 bis 80 g/m² aufweisen.

12. Vliesstoffbahn nach einem der vorhergehenden Ansprüche, dadurch gekennzeichnet, daß sie ein Flächengewicht von 50 bis 250 g/m² aufweist.

13. Zum Aufsaugen von wäßrigen Körperausscheidungen geeigneter Hygieneartikel, dadurch gekennzeichnet, daß der zur Aufnahme der wäßrigen Körperausscheidung bestimmte Teil des Hygieneartikels aus einer saugfähigen Vliesstoffbahn nach einem der vorhergehenden Ansprüche besteht.

14. Hygieneartikel nach Anspruch 13, dadurch gekennzeichnet, daß es sich um eine Damenbinde handelt.

15. Hygieneartikel nach Anspruch 13, dadurch gekennzeichnet, daß es sich um eine Babywindel handelt.

16. Hygieneartikel nach Anspruch 13, dadurch gekennzeichnet, daß es sich um einen Inkontinenzartikel handelt.

## Claims

1. Absorbent bonded fabric web, having an absorbent body capable of retaining aqueous liquids, which body consists of a layer of absorbent fibre flakes and is enclosed by two covering bonded fabric layers arranged substantially parallel to one another, wherein the covering bonded fabric layers are partly connected together,
characterised in that the individual fibre flakes forming the absorbent body consist of a spherical core, consisting of an absorbent polymer with a diameter of between 0.1 and 2 mm and polyolefin fibres connected thereto, which have a length of between 0.2 and 10 mm, the dimension achieved for the individual flakes being 0.1 to 5 mm, in particular 0.5 to 2 mm, diameter.

2. Bonded fabric web according to claim 1, characterised in that the surface weight of the absorbent layer is between 20 g/m² and 200 g/m².

3. Bonded fabric web according to one of the preceding claims, characterised in that the bonded fabric, consisting of absorbent body and covering bonded fabric layers, has a thickness of 0.2 to 30 mm.

4. Bonded fabric web according to claim 3, characterised in that the bonded fabric has a thickness of between 0.5 and 3 mm.

5. Bonded fabric web according to one of the preceding claims, characterised in that the two covering bonded fabric layers are connected together at the edges and/ or in the region lying between the edges by linear regions running substantially parallel to one another, so that the absorbent body disposed between the covering bonded fabric layers is subdivided into relatively small compartments.

6. Bonded fabric according to claim 5, characterised in that the the fibres of the covering bonded fabric layers are welded together in the connecting regions, so that a firm connection is obtained between the absorbent body and the two covering bonded fabric layers.

7. Bonded fabric web according to one of the preceding claims, characterised in that the covering bonded fabric layers are polyolefin bonded fabric layers.

8. Bonded fabric web according to claim 7, characterised in that the polyolefin is polyethylene or polypropylene.

9. Bonded fabric web according to claim 5, characterised in that in the connecting regions the two covering bonded fabric layers are fixed with chemical bonding agents.

10. Bonded fabric web according to claim 9, characterised in that the chemical bonding agents are copolymers composed of ethylene and acrylic acid, cellulose ether or polyvinyl alcohol.

11. Bonded fabric web according to one of the preceding claims, characterised in that the covering bonded fabric layers have a surface weight of 15 to 80 g/m² respectively.

12. Bonded fabric web according to one of the preceding claims, characterised in that they have a surface weight of 50 to 250 g/m^{2.}

13. Hygiene article suitable for absorbing aqueous bodily discharges, characterised in that the part of the sanitary article intended to absorb the aqueous bodily discharge consists of an absorbent bonded fabric web according to one of the preceding claims.

14. Hygiene article according to claim 13, characterised in that it is a sanitary towel.

15. Hygiene article according to claim 13, characterised in that it is a baby's napkin.

16. Hygiene article according to claim 13, characterised in that it is an article for use in the case of incontinence.

## Revendications

1. Bande d'étoffe nappée absorbante avec un corps absorbant capable de stocker des liquides aqueux, qui est constituée d'une couche de flocons fibreux absorbants et est entourée de deux couches nappées extérieures disposées essentiellement parallèles l'une à l'autre, les couches nappées extérieures étant partiellement liées entre elles,
caractérisée en ce que des flocons fibreux formant le corps absorbant sont constitués chacun d'un noyau sphérique formé dans un polymère absorbant avec un diamètre entre 0,1 et 2 mm et lié avec des fibres de polyoléfine d'une longueur entre 0,2 et 10 mm, de façon à obtenir une dimension des flocons individuels de 0,1 à 5 mm, et en particulier de 0,5 à 2 mm de diamètre.

2. Bande d'étoffe nappée selon la revendication 1 caractérisée en ce que le grammage de la couche superficielle est comprise entre 20 g/m² et 200 g/m².

3. Bande d'étoffe nappée selon l'une des revendications précédentes caractérisée en ce que l'étoffe nappée, constituée du corps absorbant et des couches nappées extérieures, présente une épaisseur de 0,2 à 30 mm.

4. Bande d'étoffe nappée selon la revendication 3 caractérisée en ce que l'étoffe nappée présente une épaisseur entre 0,5 et 3 mm.

5. Bande d'étoffe nappée selon l'une des revendications précédentes caractérisée en ce que deux couches nappées extérieures sont reliées entre elles par les bords et/ou dans les zones situées entre les bords par des zones rectilignes disposées essentiellement parallèlement les unes aux autres, de façon que le corps absorbant disposé entre les couches nappées extérieures soit divisé en petits compartiments.

6. Bande d'étoffe nappée selon la revendication 5 caractérisée en ce que dans les zones de liaison les fibres des couches nappées extérieures sont soudées entre elles, de façon à obtenir une liaison solide entre le corps absorbant et les deux couches nappées extérieures.

7. Bande d'étoffe nappée selon l'une des revendications précédentes caractérisée en ce que les couches nappées extérieures sont des couches nappées de polyoléfine.

8. Bande d'étoffe nappée selon la revendication 7 caractérisée en ce que la polyoléfine est du polyéthylène ou du polypropylène.

9. Bande d'étoffe nappée selon la revendication 5 caractérisée en ce que, dans les zones de liaison, les deux couches nappées extérieures sont solidarisées par liaisons chimiques.

10. Bande d'étoffe nappée selon la revendication 9 caractérisée en ce que les liants chimiques sont des copolymères d'éthylène et d'acide acrylique, d'éther cellulosique ou d'alcool polyvinylique.

11. Bande d'étoffe nappée selon l'une des revendications précédentes caractérisée en ce que les couches nappées extérieures présentent un grammage de 15 à 80 g/m² suivant les cas.

12. Bande d'étoffe nappée selon l'une des revendications précédentes caractérisée en ce qu'elle présente un grammage de 50 à 250 g/m².

13. Article hygiénique adapté à l'absorption de produits d'élimination corporels aqueux caractérisé en ce que la partie de l'article d'hygiène assurant l'absorption du produit d'élimination corporel aqueux est constituée d'une bande d'étoffe nappée absorbante selon l'une des revendications précédentes.

14. Article d'hygiène selon la revendication 13 caractérisé en ce qu'il s'agit d'une serviette féminine.

15. Article d'hygiène selon la revendication 13 caractérisé en ce qu'il s'agit d'une couche pour bébé.

16. Article d'hygiène selon la revendication 13 caractérisé en ce qu'il s'agit d'un article d'incontinence.
